# EUROPÄISCHE PATENTSCHRIFT

(11) **EP 1 476 418 B1**
(45) Veröffentlichungstag und Bekanntmachung des Hinweises auf die Patenterteilung: **23.06.2010**
(21) Anmeldenummer: 03706422.7
(22) Anmeldetag: 04.02.2003
(51) Int. Cl.: C07C 209/36

(54) **VERFAHREN ZUR HERSTELLUNG VON AMINEN**
METHOD FOR THE PRODUCTION OF AMINES
PROCEDE DE PRODUCTION D'AMINES

(30) Priorität: 15.02.2002 DE 10206214
(43) Veröffentlichungstag der Anmeldung: 17.11.2004
(73) Patentinhaber: BASF SE, 67056 Ludwigshafen (DE)
(72) Erfinder: ZEHNER, Peter, 67071 Ludwigshafen (DE); MÜLLER, Jörn, 49152 Bad Essen (DE)
(86) Internationale Anmeldenummer: PCT/EP2003/001063
(87) Internationale Veröffentlichungsnummer: WO 2003/068724

(56) Entgegenhaltungen:
- WO-A-00/35852
- DE-C- 19 844 901

## Beschreibung

Die Erfindung betrifft ein Verfahren zur Herstellung von Aminen durch Hydrierung der entsprechenden Nitroaromaten.

Die Herstellung von Aminen, insbesondere aromatischen Aminen, durch Hydrierung der entsprechenden Nitroaromaten ist seit langem bekannt und vielfach in der Literatur beschrieben.

Zumeist wird die Hydrierung in der Technik unter Einsatz von Hydrierkatalysatoren in der Flüssigphase durchgeführt. Dabei wird die zu reduzierende Verbindung zumeist in einem Lösungsmittel mit dem Katalysator vermischt und diskontinuierlich in einem Autoklaven oder kontinuierlich in einem Rührkessel, einem Schlaufenreaktor, einer Blasensäule oder einer Reaktorkaskade reduziert. Bei den bekannten derartigen Verfahren gibt es eine Reihe von Nachteilen, beispielsweise die Notwendigkeit des Austragens und besonders des Ausschleusens desaktivierter Katalysatoranteile, was zu Katalysatorverlusten führt. Ferner stellen die häufig auftretenden Nebenreaktionen, die zur Bildung störender Substanzen, wie z.B. teerartiger Bestandteile, und damit zu Ausbeuteminderungen führen, ein Problem vieler bislang verwendeter Verfahren dar.

In WO 00/35852 wird ein Verfahren zur Herstellung von Aminen durch Hydrierung der entsprechenden Nitroaromaten beschrieben, bei dem die Reaktion in einem vertikalen Reaktor, dessen Länge größer ist als sein Durchmesser, mit einer im oberen Bereich des Reaktors angeordneten, nach unten gerichteten Strahldüse, über die die Edukte sowie das Reaktionsgemisch zugeführt werden sowie mit einem Abzug an einer beliebigen Stelle des Reaktors, über den das Reaktionsgemisch in einem äußeren Kreislauf mittels eines Förderorgans der Strahldüse wieder zugeführt wird, sowie einer Strömungsumkehr im unteren Bereich des Reaktors durchgeführt wird, verwendet. Dieser Reaktor weist vorzugsweise innenliegende Wärmetauscher auf. Die Edukte werden vorzugsweise über die Strahldüse zugeführt, um eine möglichst gute Durchmischung zu erreichen. Bei dem in WO 00/35852 beschriebenen Verfahren können die aromatischen Amine mit einer hohen Raum-Zeit-Ausbeute und bei deutlicher Unterdrückung von Nebenreaktionen hergestellt werden.

Es hat sich allerdings gezeigt, dass im Reaktor, insbesondere in der nach unten gerichteten Strömung unterhalb der Strömungsumkehr der Flüssigphase, insbesondere in der Nähe des Reaktorausgangs, mitunter erhöhte Gehalte an Nitroaromaten auftreten, insbesondere bei Verwendung geringer Katalysatorgehalte.

In DE-C-198 44 901 wird ein Verfahren zur Herstellung von aromatischen Aminen nach dem Sumpfphasenverfahren beschrieben, bei dem die Nitroaromaten über eine Ringleitung mit Löchern in den Reaktor eingespeist werden. Die Ringleitung kann auch über einen außenliegenden Wärmeübertrager gekühlt werden, um die Gefahr einer Überhitzung und damit einer thermischen Zersetzung der Nitroaromaten auszuschließen. Bei diesem Verfahren soll eine besonders gute Verteilung der Nitroaromaten in der Reaktionsmischung erreicht werden. Als geeignete Reaktoren werden beispielsweise Loop-Reaktoren, Blasensäulen, vorzugsweise Rührkessel beschrieben. Reaktoren mit einer inneren Schlaufenströmung, wie in WO 00/35852 beschrieben, werden in diesem Dokument nicht erwähnt. Speziell bei derartigen Reaktoren kann es jedoch im Bereich unterhalb der Strömungsumkehr zu der beschriebenen Anreicherung von Nitroaromaten der flüssigen Reaktionsmischung kommen. Dies kann daran liegen, daß die Verweilzeit in der nach unten gerichteten, schnellfließenden Strömung und im Bereich der Strömungsumkehr an der Prallplatte zu gering für eine vollständige Umsetzung der Nitroaromaten ist. Da es sich bei der nach unten gerichteten Strömung um ein schnell fließendes Gemisch handelt, ist die Verweilzeit in diesem Teil des Reaktors ohnehin sehr gering. Dieser Effekt tritt insbesondere bei der Verwendung geringer Gehalte an Katalysator beziehungsweise bei Katalysatoren mit einer geringen Aktivität auf.

Aufgabe der Erfindung war es, ein Verfahren zur Herstellung aromatischen Aminen in einem Strömungsreaktor zu entwickeln, bei dem eine Katalysatordesaktivierung vermieden und die Nebenproduktbildung reduziert wird sowie am Ausgang des Reaktors keine Nitroaromaten mehr vorliegen und das mit niedrigen Katalysatorkonzentrationen betrieben werden kann.

Die Aufgabe konnte überraschenderweise dadurch gelöst werden, daß bei einem Reaktor gemäß WO 00/35852 zumindest ein Teil des Wasserstoffs und/oder der Nitroaromaten in die Flüssigphase des Reaktors so eindosiert werden, daß sie in der flüssigen Reaktionsmischung nach oben strömen.

Gegenstand der Erfindung ist demzufolge ein Verfahren zur Herstellung von Aminen durch Hydrierung der entsprechenden Nitroaromaten, in einem vertikalen Reaktor, dessen Länge größer ist als sein Durchmesser, mit einer im oberen Bereich des Reaktors angeordneten, nach unten gerichteten Strahldüse, über die das Reaktionsgemisch sowie gegebenenfalls ein Teil der Edukte zugeführt werden sowie mit einem Abzug an einer beliebigen Stelle des Reaktors, über den das Reaktionsgemisch in einem äußeren Kreislauf mittels eines Förderorgans der Strahldüse wieder zugeführt wird, sowie einer Strömungsumkehr im unteren Bereich des Reaktors, **dadurch gekennzeichnet, daß** zumindest ein Teil der eingesetzten Edukte Wasserstoff und/oder Nitroaromaten in die Flüssigphase des Reaktors so eindosiert werden, daß sie in der flüssigen Reaktionsmischung nach oben strömen.

Der Wasserstoff kann dabei sowohl in die nach oben gerichtete Strömung der flüssigen Phase des Reaktors oberhalb der Strömungsumkehr als auch in die nach unten gerichtete Strömung der flüssigen Phase des Reaktors unterhalb der Strömungsumkehr eindosiert werden, da er auf Grund seiner geringen Dichte in jedem Falle in der flüssigen Phase nach oben strömt.

Die Dosierung der Nitroaromaten erfolgt so, dass sie in die nach oben gerichtete Strömung innerhalb der Flüssigphase des Reaktors, also oberhalb der Strömungsumkehr, eindosiert werden.

In einer bevorzugten Ausführungsform der Erfindung weist der Reaktor in seinem unteren Teil eine senkrecht zur Reaktorwand angeordnete Prallplatte auf. Bei dieser Ausführungsform werden die Nitroaromaten sowie gegebenenfalls ein Teil oder der gesamte Wasserstoff oberhalb der Prallplatte in die nach oben gerichtete Strömung innerhalb des Reaktors eindosiert. In einer weiteren bevorzugten Ausführungsform der Erfindung ist im Reaktor zusätzlich ein konzentrisches Einsteckrohr parallel zur Reaktorwand angebracht, durch das die Reaktionsmischung aus der Strahldüse zu der Strömungsumkehr, insbesondere der Prallplatte, gelenkt wird.

Wie ausgeführt, kann die gesamte Menge des Wasserstoffs und der Nitroaromaten wie beschrieben dem Reaktor zugeführt werden.

Es ist ebenfalls möglich, entweder nur den Wasserstoff oder nur die Nitroaromaten an der erfindungsgemäßen Stelle in den Reaktor einzudosieren und das andere Edukt ausschließlich an einer anderen Stelle, vorzugsweise über die Strahldüse oder durch Dosierung in den äußeren Kreislauf, dem Reaktor zuzuführen. Der Wasserstoff, der nicht an der erfindungsgemäßen Stelle dem Reaktor zugeführt wird, kann auch an einer beliebigen Stelle in den Gasraum des Reaktors dosiert werden. Von dort wird er durch den Treibstrahl in die Flüssigphase des Reaktors eingeschlagen.

In einer bevorzugten Ausführungsform des erfindungsgemäßen Verfahrens wird ein Teil des zugesetzten Wasserstoffs in den äußeren Umlauf des Reaktors dosiert. Vorzugsweise wird der Wasserstoff in die Saugseite des äußeren Kreislaufs dosiert. Die Menge des in den äußeren Kreislauf dosierten Wasserstoffs sollte so bemessen sein, daß die Reaktionsmischung im äußeren Kreislauf einen Gasgehalt im Bereich zwischen 3 und 15 Gew.-%, vorzugsweise zwischen 5 und 10 Gew.-%, aufweist. In diesem Mengenbereich ist durch eine optimale Dispergierung des Gases in der flüssigen Reaktionsmischung eine ausreichende Versorgung der Reaktionsmischung mit Wasserstoff möglich. Bei höheren Gasgehalten in der Ringleitung kann es außerdem zu Problemen mit der Kreislaufpumpe kommen, geringere Gasgehalte in der Ringleitung haben keinen positiven Effekt mehr auf die Umsetzung.

Die Dosierung des Wasserstoffs an der erfindungsgemäßen Stelle kann im einfachsten Falle über ein oder mehrere Einleitungsrohre erfolgen. Eine bessere Verteilung des Wasserstoffs kann beispielsweise durch Verwendung einer Einleitungsvorriuhtung mit mehreren Öffnungen erreicht werden. Auf Grund der Bauart des für das erfindungsgemäße Verfahren eingesetzten Reaktors kommt vorzugsweise ein Verteilerring zum Einsatz. Andere bevorzugte Vorrichtungen zur Einspeisung des Wasserstoffs sind Verteilersterne oder Sinterplatten.

Die Einspeisung der Nitroaromaten kann, neben der Einspeisung an der erfindungsgemäßen Stelle, auch in den äußeren Kreislauf oder vorzugsweise in die Düse erfolgen. Die Dosierung in den äußeren Kreislauf ist weniger bevorzugt, da dort die Vermischung schlechter ist. Die Dosierung in die Düse kann in unmittelbarer Nähe des Düsenausgangs erfolgen. Eine noch bessere Durchmischung kann erreicht werden, wenn die Dosierung an dem Ende der Düse, das an der äußeren Umpumpleitung befindlich ist, oder in die äußere Umpumpleitung unmittelbar an der Düse erfolgt.

Die Dosierung der Nitroaromaten an der erfindungsgemäßen Stelle kann ebenfalls über Einleitungsrohre, Verteilersterne, Ringleitungen oder andere Vorrichtungen, die eine gute Verteilung der Nitroaromaten ermöglichen, erfolgen.

Die Dosiereinrichtung für die Nitroaromaten wird, wie ausgeführt, im unteren Teil des Reaktors oberhalb der Strömungsumkehr angebracht, wobei darauf zu achten ist, daß die Nitroaromaten in die nach oben gerichtete Strömung dosiert werden. Bevorzugt wird die Dosiereinrichtung für die Nitroaromaten zwischen der Prallplatte und dem konzentrischen Einsteckrohr angebracht.

Die Einspeisung des Wasserstoffs wird ebenfalls im unteren Teil des Reaktors so vorgenommen, daß er in der flüssigen Reaktionsmischung nach oben steigt. Besonders bevorzugt wird die Einspeisung zwischen Reaktorboden und Einsteckrohr vorgenommen.

Bei der Verwendung von Ringleitungen zur Dosierung von Wasserstoff und/oder Nitroaromaten an der erfindungsgemäßen Stelle weisen diese eine zur optimalen Verteilung ausreichende Anzahl von Bohrungen auf. Diese liegt vorzugsweise im Bereich zwischen 2 und 500, insbesondere zwischen 2 und 200. Beim Durchströmen der Bohrungen durch die Edukte tritt ein Druckverlust auf. Dieser liegt bei den Nitroaromaten vorzugsweise zwischen 0,1 und 10 bar, insbesondere zwischen 0,3 und 3 bar, beim Wasserstoff vorzugsweise zwischen 0,05 und 0,15 bar.

Um im Falle der Nitroaromaten, die sich bei den im Reaktor herrschenden Temperaturen thermisch zersetzen können, gefährliche Betriebszustände zu vermeiden, sollte der Massestrom in der Dosiereinrichtung überwacht werden. Wenn der Wert für den Massestrom abfällt, müssen die Nitroaromaten aus der Dosiervorrichtung entfernt werden. Dies kann beispielsweise durch Spülen mit warmen Wasser erfolgen. Eine weitere Möglichkeit der Reduzierung der Gefahr einer thermischen Zersetzung der Nitroaromaten besteht in der Kühlung der Dosiervorrichtung. Diese Variante ist für das erfindungsgemäße Verfahren nicht bevorzugt, da durch den Kühlmantel der Querschnitt der Dosiervorrichtung erhöht wird, was zu einer Beeinträchtigung der Strömung im Reaktor führen kann.

Abgesehen von der Dosierung der Edukte entspricht der Reaktor dem in WO 00/35852 beschriebenen Reaktor.

Die Strahldüse des Reaktors kann als Ein-, Zwei- oder Dreistoffdüse ausgelegt werden. Wenn der gesamte Wasserstoff und die gesamte Menge an Nitroaromaten am Boden zudosiert wird, ist eine Einstoffdüse bevorzugt. Bei der Einstoffdüse wird nur das flüssige Reaktionsgemisch eingedüst und der Wasserstoff und die Nitroaromaten an den bevorzugten Stellen dem Reaktor zugeführt. Vorteilhaft bei dieser Ausgestaltung ist der einfache Aufbau dieser Düse, nachteilig ist die schlechtere Dispergierung des Wasserstoffs im Reaktionsgemisch. Bei der Zwei- bzw. Dreistoffdüse, die im Aufbau aufwendiger ist, können der Wasserstoff sowie die Nitroaromaten düsenmittig oder über Ringspalte zugeführt und dispergiert werden. Bei dieser Ausgestaltung des Verfahrens ist die Dispergierung des Wasserstoffs in der Reaktionsmischung wesentlich besser.

Im Reaktor wird vorzugsweise, unabhängig von der Art der eingesetzten Nitroverbindungen, ein Druck von 5 bis 100 bar, bevorzugt 10 bis 50 bar, und eine Betriebstemperatur von 80 bis 200°C, bevorzugt 100 bis 150°C, aufrechterhalten. Der Leistungseintrag beträgt vorzugsweise an der Düse 15 bis 30 kW/1, im gesamten Reaktionssystem 3 bis 10 W/1.

Der Produktaustrag aus dem System erfolgt kontinuierlich an beliebiger Stelle. Bevorzugt erfolgt der Produktaustrag im unteren Reaktorbereich am Boden des Reaktors oder insbesondere aus der äußeren Schlaufenströmung über eine Katalysatorabtrenneinheit oder ohne eine solche. Diese Abtrenneinheit kann ein Schwerkraftabscheider, z. B. ein Settler, ein Querstromfilter oder eine Zentrifuge sein. Der Katalysator kann vom Produkt abgetrennt und anschließend in das Reaktorsystem zurückgeführt werden. Bevorzugt erfolgt der Produktaustrag unter Rückhaltung des Katalysators. Das Amin kann danach nach den üblichen und bekannten Verfahren, beispielsweise durch Destillation oder Extraktion, gereinigt werden.

Im erfindungsgemäßen Verfahren wird die Mono- und/oder Polynitroverbindung in reiner Form, als Mischung mit dem entsprechenden Mono- und/oder Polyamin, als Mischung mit dem entsprechenden Mono- und/oder Polyamin und Wasser oder als Mischung mit dem entsprechenden Mono- und/oder Polyamin, Wasser und einem insbesondere alkoholischen Lösungsmittel eingesetzt. Die aromatische Mono- und/oder Polynitroverbindung wird fein verteilt in das Gemisch eingetragen. Vorzugsweise wird die Menge an Nitroverbindung, die nicht an der erfindungsgemäßen Stelle dosiert wird, in die Strahldüse eingetragen, besonders bevorzugt in den Mischraum der Düse.

Vorzugsweise werden im erfindungsgemäßen Verfahren aromatische Nitroverbindungen mit einer oder mehreren Nitrogruppen und 6 bis 18 C-Atomen, bsp. Nitrobenzole, wie z.B. o-, m-, p-Nitrobenzol, 1,3-Dinitrobenzol, Nitrotoluole, wie z.B. 2,4-, 2,6-Dinitrotoluol, 2,4,6-Trinitrotoluol, Nitroxylole, wie z.B. 1,2-Dimethyl-3-, 1,2 Dimethyl-4-, 1,4-Dimethyl-2-, 1,3-Dimethyl-2-, 2,4-Dimethyl-1- und 1,3-Dimethyl-5-nitrobenzol, Nitronaphthaline, wie z.B. 1-, 2-Nitronaphthalin, 1,5 und 1,8-Dinitronaphthalin, Chlornitrobenzole, wie z.B. 2-Chlor-1,3-, 1-Chlor-2,4-dinitrobenzol, o-, m-, p-Chlornitrobenzol, 1,2-Dichlor-4-, 1,4-Dichlor-2-, 2,4-Dichlor-1-und 1,2-Dichlor-3-nitrobenzol, Chlornitrotoluole, wie z.B. 4-Chlor-2, 4-Chlor-3-, 2-Chlor-4- und 2-Chlor-6-nitrotoluol, Nitroaniline, wie z.B. o-, m-, p- Nitroanilin; Nitroalkohole, wie z.B. Tris(hydroxymethyl)nitromethan, 2-Nitro-2-methyl-, 2-Nitro-2-ethyl-1,3-propandiol, 2-Nitro-1-butanol und 2-Nitro-2-methyl-1-propanol sowie beliebige Gemische aus zwei oder mehreren der genannten Nitroverbindungen eingesetzt.

Bevorzugt werden nach dem erfindungsgemäßen Verfahren aromatische Nitroverbindungen, vorzugsweise Mononitrobenzol, Methylnitrobenzol oder Methylnitrotoluol, und insbesondere 2,4-Dinitrotoluol oder dessen technische Gemische mit 2,6-Dinitrotoluol, wobei diese Gemische vorzugsweise bis zu 35 Gewichtsprozent, bezogen auf das Gesamtgemisch, an 2,6-Dinitrotoluol mit Anteilen von 1 bis 4 Prozent an vicinalem DNT und 0,5 bis 1,5 % an 2,5- und 3,5-Dinitrotoluol aufweisen, zu den entsprechenden Aminen hydriert.

Insbesondere bei der Hydrierung von Dinitrotoluolisomeren zu den entsprechenden Toluylendiaminderivaten (TDA) kann das erfindungsgemäße Verfahren vorteilhaft eingesetzt werden. Die Bildung von hochmolekularen, teerartigen Nebenprodukten, die bei den Verfahren des Standes der Technik zu Ausbeuteverlusten sowie zum Verkleben und damit zur vorzeitigen Desaktivierung des Katalysators führte, konnte praktisch vollständig unterdrückt werden. Damit ist auch die Reinigung des TDA unkomplizierter als bei den Verfahren des Standes der Technik.

Die Hydrierung des Dinitrotoluols kann in Lösung durchgeführt werden. Als Lösungsmittel werden die dafür üblichen Stoffe, insbesondere niedere Alkohole, vorzugsweise Ethanol, eingesetzt. Vorzugsweise wird die Hydrierung ohne Lösungsmittel durchgeführt. Das hat die Vorteile, daß das Volumen der Reaktionsmischung geringer ist, was eine kleinere Dimensionierung des Reaktors sowie der Pumpen und Rohrleitungen ermöglicht, daß Nebenreaktionen zwischen dem Lösungsmittel und den Edukten ausgeschlossen werden sowie der Aufwand für die Aufarbeitung der Endprodukte verringert wird.

Zur Durchführung des erfindungsgemäßen Verfahrens werden die an sich bekannten Hydrierkatalysatoren für aromatische Nitroverbindungen verwendet. Es können homogene und/oder insbesondere heterogene Katalysatoren eingesetzt werden. Die heterogenen Katalysatoren werden in feinverteiltem Zustand eingesetzt und liegen in der Reaktionssuspension feinteilig suspendiert vor. Geeignete Katalysatoren sind Metalle der VIII. Nebengruppe des Periodensystems, die auf Trägermaterialien wie Aktivkohle oder Oxiden des Aluminiums, des Siliciums oder anderer Materialien aufgebracht sein können. Vorzugsweise werden Raney-Nickel und/oder geträgerte Katalysatoren auf Basis von Nickel, Palladium und/oder Platin verwendet.

Durch die Dispergierung der einzelnen Reaktanden wird in Verbindung mit den übrigen Reaktionsparametern eine intensive Durchmischung aller Komponenten, bei hohen Stoffübergangskoeffizienten und hohen volumenspezifischen Phasengrenzflächen erreicht. Die Anordnung der Kühlrohre im Reaktor parallel zu den Reaktorwänden hat eine nahezu vollständige Gradientenfreiheit des Reaktorinhalts bezüglich der Reaktionstemperatur zur Folge. Durch Vermeidung örtlicher Überhitzungen werden Nebenreaktionen deutlich zurückgedrängt und Katalysatordesaktivierung weitgehend vermieden. Es werden damit selbst bei niedrigen Katalysatorkonzentrationen hohe Raum-Zeit-Ausbeuten bei hoher Selektivität erreicht.

Wie oben beschrieben, ist das erfindungsgemäße Verfahren besonders geeignet für die Hydrierung von Dinitrotoluol zu Toluylendiamin. Gerade bei dieser Reaktion kommt es bei den Verfahren des Standes der Technik zu ausgeprägten Nebenreaktionen mit der Bildung von teerartigen Bestandteilen. Die Herstellung von Toluylendiamin nach dem erfindungsgemäßen Verfahren erfolgt bei den für die Herstellung von aromatischen Aminen üblichen, oben aufgeführten Temperaturen und Drücken.

Das erfindungsgemäße Verfahren hat mehrere Vorteile. Die Verteilung der Edukte in der Reaktionsmischung wird verbessert, was zu niedrigen Gehalten an Nebenprodukten und geringer Desaktivierung des Katalysators führt. Die Umsetzung der Nitroaromaten erfolgt vollständiger, das heisst die Konzentration von Nitroaromaten am Ausgang des Reaktors sinkt deutlich. Dies bietet die Möglichkeit die Raum-Zeit-Ausbeute im Reaktor zu erhöhen. Im Vergleich mit dem in WO 00/35852 beschriebenen Verfahren erfolgt noch einmal eine deutliche Reduzierung der Menge der Nitroaromaten im Endprodukt. Überraschenderweise wird die auch Alterung des Katalysators vermindert. Die Verweilzeit der Nitroaromaten im Reaktor wird erhöht, dadurch kann prinzipiell auch die Katalysatorkonzentration vermindert werden.

Durch die erfindungsgemäße Dosierung des Wasserstoffs kann der Energieeintrag in den Reaktor verringert werden, da der Wasserstoff den Gasgehalt und somit die Stofftransportleistung im Ringraum des Reaktors erhöht und durch seine Auftriebskraft die interne Schlaufenströmung stabilisiert. Der Leistungseintrag durch die Strahldüse und damit auch die Menge des äußeren Kreislaufs kann deutlich verringert werden. Neben der Energieeinsparung führt dies auch zu einer geringeren mechanischen Beanspruchung und somit zu einer längeren Lebensdauer des Katalysators.

Die Erfindung soll an dem nachfolgenden Beispiel näher beschrieben werden.

### Beispiel 1

Es wird ein zylinderförmiger Reaktor mit einem außenliegenden Kreislauf mit Pumpe, einer Düse, einer Prallplatte im unteren Reaktorteil sowie einem konzentrischen Einsteckrohr eingesetzt. Das Reaktionsvolumen des Reaktors beträgt rund 12 m³. Der Reaktor ist mit 350 parallel geschalteten Fieldrohren (4) versehen, die insgesamt einer Kühlfläche von etwa 300 m² entsprechen. Die Menge des in die Fieldrohre eingespeisten Kühlwassers betrug 250 m³/h, die Temperatur des in die Fieldrohre eingespeisten Kühlwassers lag bei 50°C.

Über einen Verteilerring mit 12 Austrittsöffnungen mit einem Durchmesser von je 5 mm wurden 7 t/h einer Dinitrotoluolschmelze, in die flüssige Reaktionsmischung oberhalb der Prallplatte eingeleitet. Durch gleichzeitiges Einleiten von 5200 Nm³/h Wasserstoff in den Gasraum des Reaktors wurde im Reaktor ein Druck von 25 bar aufrechterhalten. Durch einen unterhalb der Prallplatte angebrachten Verteilerring mit 30 Bohrungen mit einem Durchmesser von je 8 mm wurde Wasserstoff eingeleitet. Zur Aufrechterhaltung der Schlaufenströmung wurde im externen Produktkreislauf ein Volumenstrom von 500 m³/h umgewälzt. In der Reaktionsdüse herrschte ein Überdruck gegenüber dem Gasraum des Reaktors von rund 2,5 bar, der Leistungseintrag betrug 3,5 kW/m³. Die Reaktion verlief unter nahezu isothermen Bedingungen, da die entstehende Reaktionswärme bereits am Ort ihrer Entstehung abgeführt wurde. Die maximale Reaktionstemperatur im unteren Drittel des Reaktors betrug 125°C. Dem Reaktor wurden gleichzeitig unter Rückhaltung des Katalysators 4,64 t/h eines entsprechenden Diaminotoluolgemisches sowie 2,74 t/h Wasser über einen im äußeren Kreislauf installierten Querstromfilter kontinuierlich entnommen, was einer Raum-Zeit-Ausbeute von 400 kg Amingemisch/(m³·h) entsprach. Die Ausbeute an Diamin betrug, bezogen auf eingesetztes Dinitrotoluol, > 99 %. Bei der destillativen Aufarbeitung fielen 0,15 % niedrigsiedende Nebenprodukte ("Leichtsieder") und 0,75 % teerartige Produkte ("Hochsieder") an. Der Gehalt an Nitro- bzw. Aminonitroverbindungen im Produktaustrag lag unterhalb der Nachweisgrenze von 10 ppm. Eine merkliche Desaktivierung des eingesetzten Hydrierkontakts konnte für den oben beschriebenen Betriebszustand auch nach 100 h Reaktionszeit nicht festgestellt werden.

## Patentansprüche

1. Verfahren zur Herstellung von Aminen durch Hydrierung der entsprechenden Nitroaromaten, in einem vertikalen Reaktor, dessen Länge größer ist als sein Durchmesser, mit einer im oberen Bereich des Reaktors angeordneten, nach unten gerichteten Strahldüse, über die das Reaktionsgemisch sowie gegebenenfalls ein Teil der Edukte zugeführt werden sowie mit einem Abzug an einer beliebigen Stelle des Reaktors, über den das Reaktionsgemisch in einem äußeren Kreislauf mittels eines Förderorgans der Strahldüse wieder zugeführt wird, sowie einer Strömungsumkehr im unteren Bereich des Reaktors durchgeführt wird, **dadurch gekennzeichnet, daß** zumindest ein Teil der eingesetzten Edukte Wasserstoff und/oder Nitroaromaten in die Flüssigphase des Reaktors so eindosiert werden, daß sie in der Flüssigphase nach oben steigen.

2. Verfahren nach Anspruch 1, **dadurch gekennzeichnet, daß** die Nitroaromaten oberhalb der Strömungsumkehr zudosiert werden.

3. Verfahren nach Anspruch 1, **dadurch gekennzeichnet, daß** der Reaktor in seinem unteren Teil eine senkrecht zur Reaktorwand angeordnete Prallplatte aufweist.

4. Verfahren nach Anspruch 1 oder 2, **dadurch gekennzeichnet, daß** im Reaktor ein konzentrisches Einsteckrohr parallel zur Reaktorwand angebracht ist.

5. Verfahren nach einem der Ansprüche 1 bis 3, **dadurch gekennzeichnet, daß** die Nitroaromaten zwischen Prallplatte und Einsteckrohr in den Reaktor dosiert werden.

6. Verfahren nach einem der Ansprüche 1 bis 4, **dadurch gekennzeichnet, daß** der Wasserstoff zwischen Reaktorboden und Einsteckrohr dosiert wird.

7. Verfahren nach einem der Ansprüche 1 bis 5, **dadurch gekennzeichnet, daß** ein Teil des eingesetzten Wasserstoffs in den äußeren Kreislauf des Reaktors dosiert wird.

8. Verfahren nach einem der Ansprüche 1 bis 6, **dadurch gekennzeichnet, daß** soviel Wasserstoff in den äußeren Kreislauf des Reaktors dosiert wird, daß die Reaktionsmischung im äußeren Kreislauf einen Gasgehalt im Bereich zwischen 3 und 15 Gew.-% aufweist.

## Claims

1. A process for preparing amines by hydrogenation of the corresponding nitroaromatics in a vertical reactor whose length is greater than its diameter and which has in its upper region a downward-directed jet nozzle via which the reaction mixture and, if desired, part of the starting materials are introduced, has an outlet at any point on the reactor via which the reaction mixture is conveyed in an external circuit by means of a transport device back to the jet nozzle and has a flow reversal in its lower region, wherein at least part of the hydrogen and/or nitroaromatic starting materials used is fed into the liquid phase of the reactor in such a way that they travel upward in the liquid phase.

2. The process according to claim 1, wherein the nitroaromatics are introduced above the flow reversal.

3. The process according to claim 1, wherein the reactor has an impingement plate arranged perpendicular to the reactor wall in its lower part.

4. The process according to claim 1 or 2, wherein a concentric plug-in tube is installed in the reactor parallel to the reactor wall.

5. The process according to any of claims 1 to 3, wherein the nitroaromatics are fed into the reactor between impingement plate and plug-in tube.

6. The process according to any of claims 1 to 4, wherein the hydrogen is fed in between the bottom of the reactor and the plug-in tube.

7. The process according to any of claims 1 to 5, wherein part of the hydrogen used is fed into the external circuit of the reactor.

8. The process according to any of claims 1 to 6, wherein hydrogen is fed into the external circuit of the reactor in such an amount that the reaction mixture in the external circuit has a gas content in the range from 3 to 15% by weight.

## Revendications

1. Procédé pour la production d'amines par hydrogénation des composés aromatiques nitrés correspondants, dans un réacteur vertical dont la longueur est supérieure à son diamètre, comportant disposée dans la zone supérieure du réacteur une buse de projection dirigée vers le bas, par laquelle sont introduits le mélange réactionnel ainsi qu'éventuellement une partie des produits de départ, ainsi qu'en un point quelconque du réacteur un conduit de décharge, par lequel le mélange réactionnel est renvoyé à la buse de projection au moyen d'un organe de transport dans un circuit extérieur, ainsi qu'une inversion de courant qui est effectuée dans la zone inférieure du réacteur, **caractérisé en ce qu'**au moins une partie des produits de départ utilisés hydrogène et/ou composés aromatiques nitrés, sont introduits par addition dosée dans la phase liquide du réacteur, de sorte qu'ils montent vers le haut dans la phase liquide.

2. Procédé selon la revendication 1, **caractérisé en ce que** les composés aromatiques nitrés sont introduits par addition dosée au-dessus de l'inversion de courant.

3. Procédé selon la revendication 1, **caractérisé en ce que** le réacteur comporte dans sa partie inférieure une chicane déflectrice disposée perpendiculairement à la paroi du réacteur.

4. Procédé selon la revendication 1 ou 2, **caractérisé en ce que** dans le réacteur un tube concentrique inséré est placé parallèlement à la paroi du réacteur.

5. Procédé selon l'une quelconque des revendications 1 à 3, **caractérisé en ce que** les composés aromatiques nitrés sont introduits directement dans le réacteur entre chicane déflectrice et tube inséré.

6. Procédé selon l'une quelconque des revendications 1 à 4, **caractérisé en ce que** l'hydrogène est introduit par addition dosée entre fond du réacteur et tube inséré.

7. Procédé selon l'une quelconque des revendications 1 à 5, **caractérisé en ce qu'**une partie de l'hydrogène utilisé est introduit par addition dosée dans le circuit extérieur du réacteur.

8. Procédé selon l'une quelconque des revendications 1 à 6, **caractérisé en ce qu'**une quantité suffisante d'hydrogène est introduite par addition dosée dans le circuit extérieur du réacteur pour que le mélange réactionnel présente dans le circuit extérieur une teneur en gaz dans la plage comprise entre 3 et 15 % en poids.
